# EUROPEAN PATENT SPECIFICATION

(11) **EP 3 318 254 B1**
(45) Date of publication and mention of the grant of the patent: **21.04.2021**
(21) Application number: 16821568.9
(22) Date of filing: 27.06.2016
(51) Int. Cl.: A61K 31/20, A23L 33/105, A23L 33/115, A61P 3/04

(54) **AZELAIC ACID COMPOSITION HAVING ADIPOSE TISSUE TRIGLYCERIDE DEGRADATION EFFECT**
AZELAINSÄUREZUSAMMENSETZUNG MIT TRIGLYCERIDABBAUEFFEKT IN FETTGEWEBE
COMPOSITION D'ACIDE AZÉLAÏQUE AYANT UN EFFET DE DÉGRADATION DES TRIGLYCÉRIDES DU TISSU ADIPEUX

(30) Priority: 03.07.2015 KR 20150095529
(43) Date of publication of application: 09.05.2018
(73) Proprietor: Korea University Research and Business Foundation, Seoul 02841 (KR)
(72) Inventor: LEE, Sung-Joon, Seoul 06070 (KR)
(74) Representative: dompatent von Kreisler Selting Werner - Partnerschaft von Patent- und Rechtsanwälten mbB
(86) International application number: PCT/KR2016/006859
(87) International publication number: WO 2017/007162

(56) References cited:
- KR-A- 20040 010 681
- KR-A- 20120 064 604
- KR-A- 20130 101 515
- MUTHULAKSHMI SHANMUGAM ET AL: "Efficacy of azelaic acid on hepatic key enzymes of carbohydrate metabolism in high fat diet induced type 2 diabetic mice", BIOCHIMIE, vol. 95, no. 6, 9 February 2013 (2013-02-09), pages 1239-1244, XP028593691, ISSN: 0300-9084, DOI: 10.1016/J.BIOCHI.2013.01.018
- LEANNE WILSON-FRITCH ET AL: "Mitochondrial remodeling in adipose tissue associated with obesity and treatment with rosiglitazone", JOURNAL OF CLINICAL INVESTIGATION, vol. 114, no. 9, 1 November 2004 (2004-11-01), pages 1281-1289, XP055545210, GB ISSN: 0021-9738, DOI: 10.1172/JCI200421752
- MYLÈNE PERREAULT ET AL: "PPAR-delta Agonism for the Treatment of Obesity and Associated Disorders: Challenges and Opportunities", PPAR RESEARCH, vol. 2008, 1 January 2008 (2008-01-01), pages 1-9, XP055544931, US ISSN: 1687-4757, DOI: 10.1155/2008/125387
- KANG, N. ET AL.: 'Olfactory Receptor Olfr544 Responding to Azelaic Acid Regulates Glucagon Secretion in a-cells of Mouse Pancreatic Islets' BIOCHEMICAL AND BIOPHYSICAL RESEARCH COMMUNICATIONS vol. 460, no. 3, 21 March 2015, pages 616 - 621, XP029156536
- MUTHULAKSHMI, S. ET AL.: 'Efficacy of Azelaic Acid on Hepatic Key Enzymes of Carbohydrate Metabolism in High Fat Diet Induced Type 2 Diabetic Mice' BIOCHIMIE vol. 95, no. 6, 28 February 2013, pages 1239 - 1244, XP028593691

## Description

### [Techmical Field]

The present invention relates to a pharmaceutical composition or functional food containing azelaic acid as an active ingredient, and more particularly, to a pharmaceutical composition or health functional food containing azelaic acid having an effect of promoting triglyceride hydrolysis in adipose tissue and reducing accumulation of the triglycerides through a cAMP-PKA-HSL signaling pathway by binding to an olfactory receptor 544 (Olfr544), which is a G-protein coupled receptor (GPCR) expressed in a membrane of the adipose tissue, to activate Olfr544 as an active ingredient.

### [Background Art]

Excessive accumulation of energy in the body causes various metabolic diseases. Obesity, which is a representative disease caused by destruction of the balance of body energy metabolism, leads to insulin resistance and results in other diseases such as hyperlipidemia, hypertension, and the like. It is known that in order to suitably maintain body energy metabolism, it is essential to adjust life-style, and in the case of increasing an exercise amount and decreasing food intake through a suitable diet, obesity may be treated to some extent.

It is most important to induce changes in diet and life-style, including exercise, but in most of chronic disease patients, it is difficult to treat obesity only by modifying the lifestyle, management including drug therapy using a lipid lowering agent is required. As a result of studies for treating obesity, several drugs have been developed and used, but in the case of materials suppressing appetite by acting on the central nervous system, for example, fenfluramine/phentermine, or sibutramine, there are problems in that commercialized drugs were withdrawn from the market due to severe side effects such as development of cardiovascular diseases, and the like. Further, in the case of orlistat corresponding to an oral lipase inhibitor, orlistat is not effective for Koreans, and the like, who do not eat high-fat diets. Therefore, since efficacy of existing drugs is not high, there is a need to develop a novel material. Meanwhile, since there are many people who are reluctant to take drugs and are burdened due to taking drugs, a necessity for research into an active ingredient capable of treating or preventing obesity or chronic diseases by using a safe food ingredient or a material produced in the body has increased MUTHULAKSHMI SHANMUGAM ET AL: "Efficacy of azelaic acid on hepatic key enzymes of carbohydrate metabolism in high fat diet induced type 2 diabetic mice", BIOCHIMIE, vol. 95, no. 6 , pages 1239-1244 discloses that azelaic acid prevents body weight gain in diabetic mice fed with a high-fat diet. MYLENE PERREAULT ET AL: "PPAR-delta Agonism for the Treatment of Obesity and Associated Disorders: Challenges and Opportunities", PPAR RESEARCH, vol. 2008, 1 January 2008 (2008-01-01), pages 1-9 discloses that PPARdelta agonists induce body weight loss in obese mice.

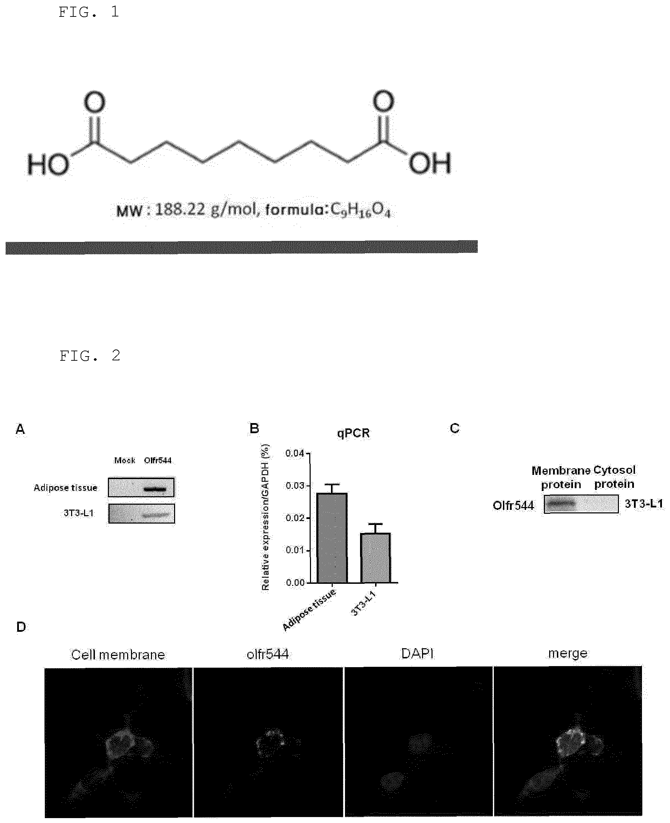

A chemical name of azelaic acid is nonanedioic acid corresponding to a dicarboxylic acid having 9 carbon atoms, and azelaic acid is formed in an omega-oxidation process or formed as a peroxide of linoleic acid in the body. Alternatively, azelaic acid is also formed as a natural material in various grains such as wheat, barley, oatmeal, sorghum, and the like, to thereby be ingested in a form of food (FIG. 1). According to results of studies conducted up to now, it is known that azelaic acid is effective for inflammatory skin diseases such as flushing, acne, and the like, and contents of research into effects of azelaic acid on arteriosclerosis, blood glucose control, an anti-cancer effect thereof, and the like, have been partially reported, but research into a detailed mechanism thereof such as identification of a target protein, and the like, has not been conducted. Research into azelaic acid associated with an effect of reducing body fat has not been reported, and associated mechanism of action was not studied.

Olfr544 is an olfactory receptor (hereinafter, referred to as 'Olfr544'), and generally, it was known that the olfactory receptor is expressed in the olfactory epithelial cells to serve to transmit smell information to the cerebrum. However, according to the recent report, it is known that Olfr544 is expressed in various tissue as well as the olfactory tissue. 400 or more olfactory receptor genes are present in humans, which correspond to 1 to 2% of human genome, and it is not surprising that the olfactory receptor occupying a large portion of the genome performs functions required in general tissue cells as well as olfactory information transmission.

According to results of a recent study, various functions of the olfactory receptor in general tissue were reported. That is, research into an olfactory receptor having a function of detecting a pheromone material in the sperm, OR2AT4 participating in regeneration of skin in the skin keratinocytes, and Olfr78 expressed in the kidney tissue to regulate secretion of renin hormone, and the like, has been reported. Recently, the present inventors reported that OR1A1 is expressed in the liver tissue to regulate triglyceride metabolism in the liver tissue (Wu C et al., Int. J. Biochem. Cell Biol., 64:75-80, 2015), and various researchers have reported novel functions of the olfactory receptor.

Based on this technical background, the present inventors tried to develop a plant derived material containing azelaic acid capable of improving lipid metabolism (alleviating obesity) by effectively decreasing a content of triglycerides in adipose tissue to decrease body fat. As a result, the present inventors found that azelaic acid suppresses lipid accumulation of the adipose tissue by acting as a ligand of Olfr544, which is an olfactory receptor ectopically expressed in 3T3-L1 adipocytes, to activate a cAMP-PKA-HSL signaling pathway, thereby completing the present invention.

### [Disclosure]

An object of the present invention is to provide a composition for improving lipid metabolism in adipose tissue (composition for alleviating obesity), containing azelaic acid having an effect of promoting triglyceride hydrolysis in the adipose tissue to effectively reduce accumulation of body fat.

According to an aspect of the present invention, there is provided a pharmaceutical composition for treating obesity, containing azelaic acid as an active ingredient.

According to another aspect of the present invention, there is provided a health functional food for alleviating obesity, containing azelaic acid as an active ingredient.

According to another aspect of the present disclosure, there is provided a method of treating, or alleviating obesity by administering azelaic acid.

According to another aspect of the present disclosure, there is provided uses of azelaic acid for manufacturing medicament for treating, or alleviating obesity.

### [Brief Description of Drawings]

FIG. 1 illustrates a chemical structure, a molecular formula, and a molecular weight of azelaic acid (trans azelaic acid).
FIGS. 2A to 2D illustrate that Olfr544 corresponding to a target molecule of azelaic acid is significantly expressed in 3T3-L1 adipocytes and mouse adipose tissue, wherein FIG. 2A illustrates a result by confirming expression of Olfr544 through a reverse transcription-polymerase chain reaction (RT-PCR) experiment, FIG. 2B illustrates a result by confirming expression of Olfr544 through a quantitative real-time polymerase chain reaction (qPCR) experiment, FIG. 2C illustrates a result by confirming expression of Olfr544 through a western blot experiment, and FIG. 2D illustrates a result by confirming expression of Olfr544 through an immunohistochemistry experiment.

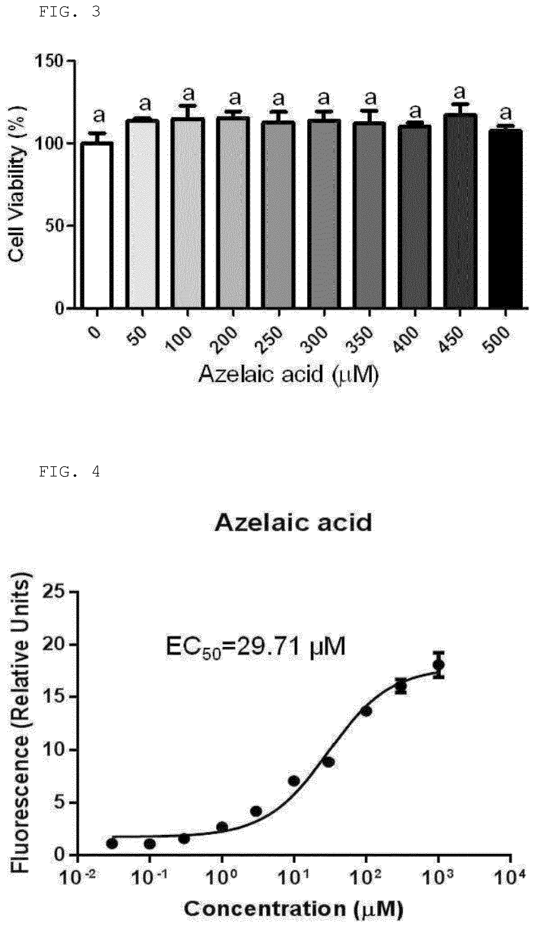

FIG. 3 illustrates a result obtained by measuring cytotoxicity of azelaic acid in 3T3-L1 adipocytes by MTT assay.
FIG. 4 illustrates a result obtained by performing reporter gene assay after introducing an Olfr544 expression vector and a CRE-Luciferase reporter vector into a Hana3A culture cell line corresponding to a cell line in which expression efficiency of an olfactory receptor is increased in order to detect an effect (activity) of azelaic acid as an Olfr544 ligand. An EC₅₀ value means a concentration of an agonist corresponding to 50% of maximum efficiency.

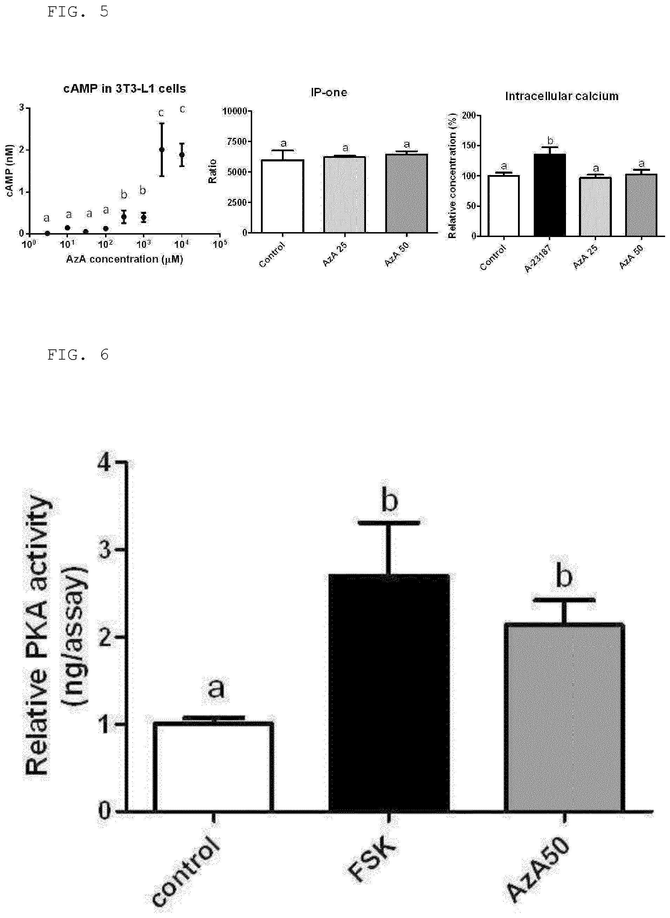

FIGS. 5A to 5C illustrate results obtained by measuring concentrations of cAMP (FIG. 5A), IP-one (metabolite of IP₃, FIG. 5B), and intracellular calcium (FIG. 5C), which are main second messengers participating in intracellular signal transduction, after treating 3T3-L1 adipocytes with azelaic acid (AzA).
FIG. 6 illustrates a result obtained by measuring a protein kinase A (PKA) activity at the time of treating 3T3-L1 adipocytes with azelaic acid (AzA) for 30 minutes. control, negative control; 1µM FSK(forskolin), positive control; 50µM AzA (Azelaic Acid). Different alphabet letters indicate statistically significant differences (p < 0.05) through analysis of variance (ANOVA) in Tukey's test.

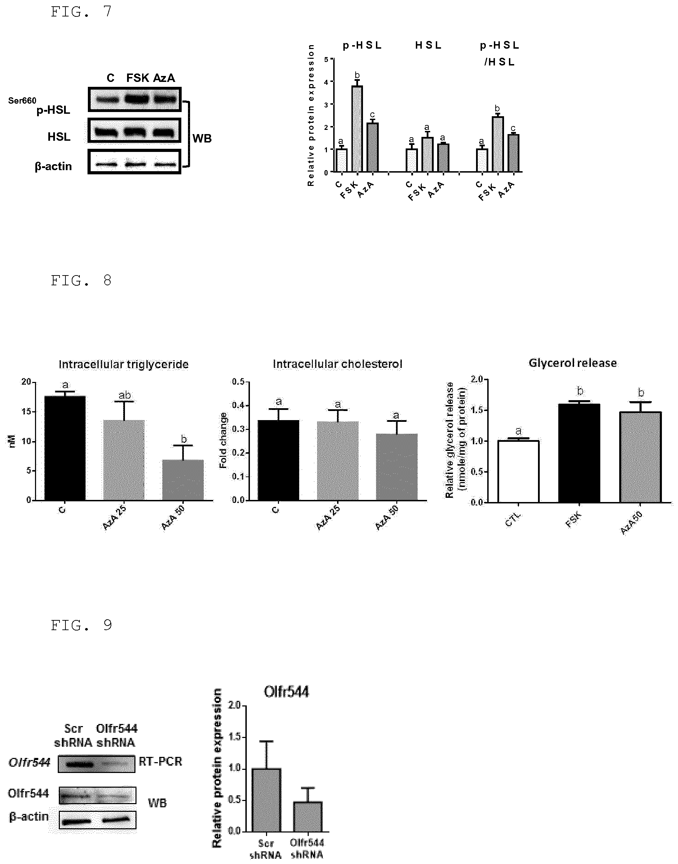

FIG. 7 illustrates a result of confirming presence or absence of phosphorylation (degrees of phosphorylation) of cAMP-response element binding protein (CREB) and hormone-sensitive lipase (HSL) corresponding to target proteins of PKA at the time of treating 3T3-L1 adipocytes with azelaic acid (AzA) for 2 hours. C (control), negative control; 1µM FSK(forskolin), positive control; 50µM AzA (Azelaic Acid).
FIGS. 8A to 8C illustrate results obtained by measuring concentrations of intracellular lipids and an amount of glycerol released by triglyceride hydrolysis at the time of treating 3T3-L1 adipocytes with azelaic acid (AzA) for 2 hours. FIG. 8A illustrates a concentration of intracellular triglyceride, FIG. 8B illustrates a concentration of intracellular cholesterol, and FIG. 8C illustrates a concentration of glycerol released from the cells. C (control), negative control; 1µM FSK(forskolin), positive control; 50µM AzA (Azelaic Acid). Different alphabet letters indicate statistically significant differences (p < 0.05) through analysis of variance (ANOVA) in Tukey's test.
FIGS. 9A and 9B illustrate results obtained by confirming an effect of inhibiting expression of Olfr544 genes at the time of transfecting 3T3-L1 adipocytes with Olf544 shRNA through a RT-PCR experiment (FIG. 9A) and a western blot experiment (FIG. 9B).

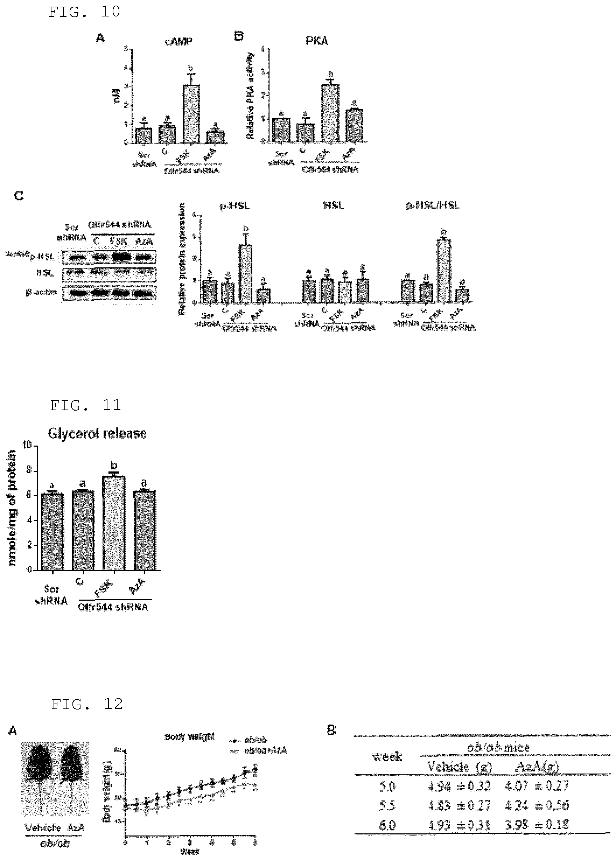

FIGS. 10A to 10C illustrate results obtained by confirming a concentration of intracellular cAMP (FIG. 10A), PKA activity (FIG. 10B), and presence or absence of phosphorylation (degrees of phosphorylation) of HSL (FIG. 10C) at the time of treating 3T3-L1 adipocytes in which the Olfr544 gene was knocked-down with azelaic acid (AzA) for 2 hours.
FIG. 11 illustrates a result obtained by measuring an amount of glycerol released due to a lipolysis effect at the time of treating 3T3-L1 adipocytes in which the Olfr544 gene was knocked-down with azelaic acid (AzA) for 2 hours.
FIGS. 12A and 12B illustrate results obtained by confirming changes in body weight (FIG. 12A) and feed intake amounts (FIG. 12B) after orally administering azelaic acid (AzA) into genetically obesity-induced *ob*/*ob* mice at a concentration of 50mg/kg for 6 weeks.

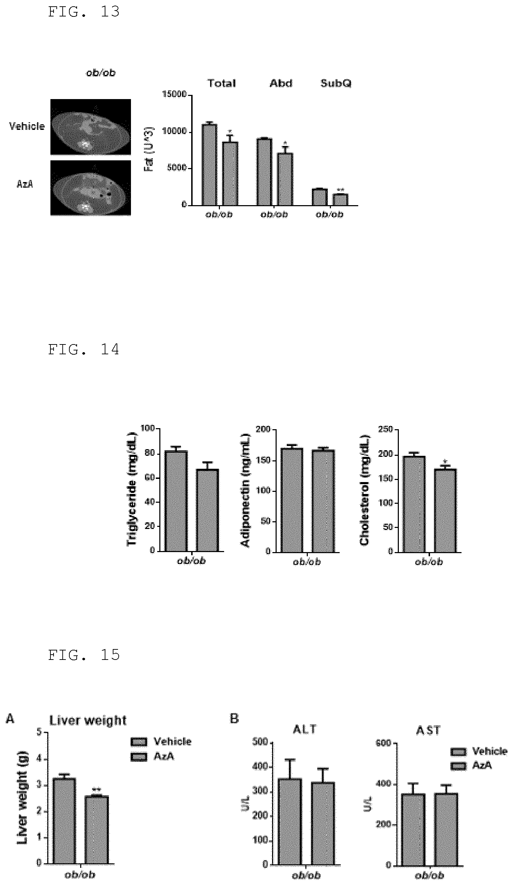

FIG. 13 illustrates results obtained by measuring total fat (Total), abdominal fat (Adb), and subcutaneous fat (SubQ) using a micro-CT method after orally administering azelaic acid (AzA) into genetically obesity-induced *ob*/*ob* mice at a concentration of 50mg/kg for 6 weeks.
FIGS. 14A to 14C illustrate results obtained by measuring concentrations of triglyceride (FIG. 14A), adiponectin (FIG. 14B), and cholesterol (FIG. 14C) in the blood after orally administering azelaic acid (AzA) into genetically obesity-induced *ob*/*ob* mice at a concentration of 50mg/kg for 6 weeks.
FIGS. 15A to 15C illustrate results obtained by measuring a weight of the liver tissue (FIG. 15A) and levels of alanine aminotransferase (ALT) and aspartate aminotransferase (AST) (FIGS. 15B and 15C) which are hepatotoxicity indexes, after orally administering azelaic acid (AzA) into genetically obesity-induced *ob*/*ob* mice at a concentration of 50mg/kg for 6 weeks.

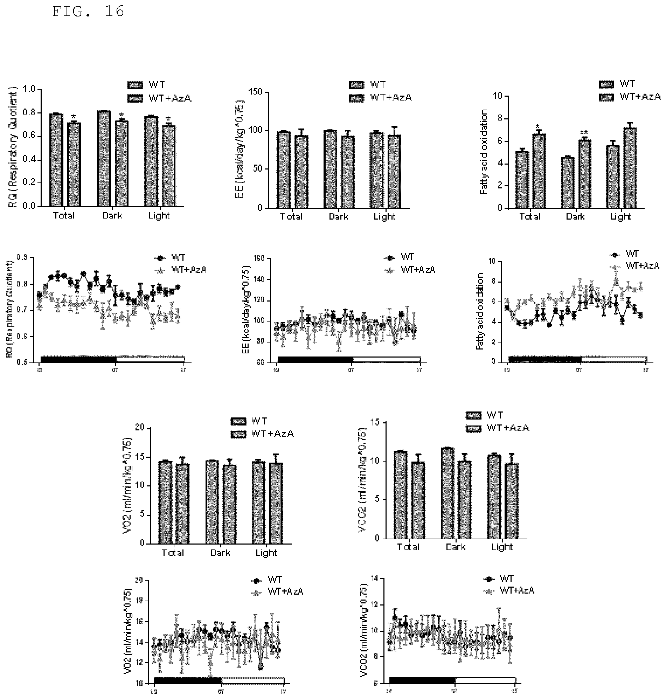

FIG. 16 illustrates results obtained by measuring a total energy expenditure (EE), a respiratory quotient (RQ), fatty acid oxidation, a consumption amount of O₂, and a production amount of CO₂ using indirect carlorimetry after orally administering azelaic acid (AzA) into normal mice at a concentration of 50mg/kg for 6 weeks.

### [Detailed Description of the Embodiments]

Unless otherwise defined herein, all of the technical and scientific terms used in the present specification have the same meanings as those understood by specialists skilled in the art to which the present invention pertains. Generally, nomenclature used in the present specification is well known and commonly used in the art.

In the present invention, it was confirmed that at the time of treating 3T3-L1 adipocytes with azelaic acid, azelaic acid had a triglyceride hydrolysis promotion effect and a triglyceride accumulation reduction effect by activating an olfactory receptor Olfr544. More specifically, in order to evaluate an adipose triglyceride accumulation suppression effect of azelaic acid, a content of triglycerides in the 3T3-L1 adipocytes treated with azelaic acid and a release amount of glycerol corresponding to a product of triglyceride hydrolysis were analyzed, thereby evaluating a triglyceride hydrolysis effect in the 3T3-L1 adipocytes. Further, in the present invention, a mechanism of the triglyceride hydrolysis effect of azelaic acid in a c-AMP-PKA-HSL signaling pathway by Olfr544 was identified by confirming characteristics of azelaic acid as an Olfr544 agonist in the 3T3-L1 adipocytes and a Hana3A cell line in which Olfr544 is expressed.

In the present invention, as a result of orally administering azelaic acid corresponding to the Olfr544 agonist to *ob*/*ob* mice corresponding to genetically obesity-induced mice for 6 weeks, it was confirmed that body fat was decreased, this was resulted from an increase in lipid oxidation in a bio-metabolic rate experiment.

Azelaic acid is naturally formed in a human body or is a food ingredient present in grains such as barley, oats, rye, and the like, which people mainly eat as food. According to reported results of a study, a concentration of azelaic acid in a human body is about 10 to 50µM, and it is widely known that generally, azelaic acid may be safely ingested. Further, in a MTT toxicity test, even in a case of treating 3T3-L1 adipocytes with azelaic acid at a concentration of up to 500µM, it was confirmed that toxicity was not exhibited.

Therefore, the present invention relates to a pharmaceutical composition for treating obesity, containing azelaic acid as an active ingredient.

In another aspect, the present disclosure relates to a method of treating, or alleviating obesity by administering azelaic acid to a subject.

In another aspect, the present disclosure relates to uses of azelaic acid for manufacturing medicament for treating, or alleviating obesity.

In the present invention, treatment of obesity may mean a decrease in triglyceride in adipose tissue, suppression of lipid accumulation in the adipose tissue, a decrease in body weight, or a decrease in body fat. Azelaic acid may increase activities of an olfactory receptor (OR), wherein the olfactory receptor may be an olfactory receptor 544 (Olfr544). Further, azelaic acid may promote triglyceride hydrolysis in the adipose tissue.

It is known that accumulation of fat (or lipid) and excessive energy in the adipose cells may cause obesity, and is a main cause of diabetes mellitus, hyperlipidemia, fatty liver, arteriosclerosis, hypertension, and cardiovascular diseases, which are various lipid-associated metabolic diseases, or metabolic syndrome in which the above-mentioned diseases are simultaneously and multiply occurred, etc. Metabolic syndrome indicates a syndrome in which risk factors such as hyperlipidemia, hypertension, glucose metabolism disorder, and obesity are simultaneously exhibited. Recently, this syndrome was officially named as metabolic syndrome or insulin resistance syndrome through an adult treatment program III (ATP III) made by the World Health Organization and National Heart, Lung, and Blood Institute (NHLBI) of the National Institute of Health (NIH).

The term "fatty liver" used herein means a state in which fat is excessively accumulated in liver cells due to fat metabolism disorder in the liver, which causes various diseases such as angina, myocardial infarction, stroke, arteriosclerosis, fatty liver, pancreatitis, and the like.

In the present invention, azelaic acid obtained by juice extraction, hot water extraction, ultrasonic extraction, solvent extraction, reflux extraction may be used, wherein the solvent extraction may be extraction using ethanol, methanol, acetone, hexane, ethyl acetate, methylene chloride, water, or a mixture thereof. The azelaic acid may be extracted by various methods generally used to prepare natural azelaic acid.

The term "azelaic acid" used herein may be used interchangeably with nonanedioic acid corresponding to a synonym.

Since the azelaic acid according to the present invention is a natural material, azelaic acid does not have toxicity, such that a large amount of azelaic acid may be continuously used as a drug.

The composition containing azelaic acid according to the present invention may be formulated together with or combined with drugs such as an anti-histamine, an analgesic, an anti-cancer agent, an antibiotic, and the like, which are already used.

Unless otherwise described, the term "treat" used herein means that a disease to which the term is applied, one or more symptoms of the disease is reversed or alleviated, or progress of the disease is suppressed or prevented in a subject having the disease. As used herein, the term "treatment" means a treating behavior when the term "treat" is defined as described above.

The term "pharmaceutical composition" or "medicinal composition" means a mixture of a compound containing azelaic acid according to the present invention and other chemical ingredients such as a diluent or a carrier.

The term "physiologically acceptable" is defined as a carrier or diluent that does not damage biological activities and physical properties of the compound.

The term "carrier" or "vehicle" is defined as a compound allowing a target compound to be easily introduced to cells or tissue. For example, dimethylsulfoxide (DMSO) is a carrier generally used to allow various organic compounds to be easily introduced into cells or tissue of living organisms.

The term "diluent" is defined as a compound stabilizing a biologically active form of a target compound and diluted in water dissolving the compound. A salt dissolved in a buffer solution is used as a diluent in the art. A generally used buffer solution is phosphate buffered saline. The reason is that the phosphate buffer saline is an imitation of a salt form of a body fluid. Since a buffer solution may control a pH of a solution at a low concentration, it is rare for a buffer diluent to change biological activity of a compound.

Each of the compounds containing azelaic acid, used in the present specification may be administered to human patient as it is, or be administered as a pharmaceutical composition in which the compound is mixed with other active ingredients or a suitable carrier or excipient as in a combination therapy.

A medicinal composition or pharmaceutical composition suitable for being used in the present invention includes a composition in which active ingredients are contained in amounts enough to achieve the desired objects. More specifically, a therapeutically effective amount means an amount of a compound effective for extending survival of an object to be treated, reducing, or alleviating symptoms of diseases. Particularly, in view of detailed contents of the disclosure provided herein, the therapeutically effective amount may be determined by those skilled in the art.

A therapeutically effective amount of the compound containing azelaic acid according to the present invention may be initially measured from cell culture analysis. For example, a dose may be calculated in an animal model in order to obtain a circulating concentration range including a half maximal inhibitory concentration (IC₅₀) or half maximal effective concentration (EC₅₀) determined in cell culture. This information may be used to more accurately determine a useful dose in human.

An administration dose of the azelaic acid may be changed within the above-mentioned range depending on an adopted administration formulation and a used administration route. Accurate calculation, the administration route, and the administration dose may be selected by individual doctors in consideration of states of a patient (for example, see Fingl et al., 1975, "The Pharmacological Basis of Therapeutics", Ch. 1, p. 1).

A preferable administration dose of azelaic acid according to the present invention may be changed according to a state and weight of a patient, a degree of disease, a drug formulation, and an administration route and duration, but be appropriately selected by those skilled in the art. Generally, a dose range of the composition administrated to a patient may be about 0.5 to 1000 mg/kg based on a body weight of a patient. However, in order to obtain a preferable effect, the azelaic acid according to the present invention may be administered at a daily dose of 0.0001 to 200mg/kg, preferably 0.001 to 100mg/kg. One dose may be administered once a day, or divided into several doses and then administered. Therefore, the scope of the present invention is not limited to the administration dose.

Azelaic acid according to the present invention may be administered to mammals such as rats, mice, livestock, humans, and the like, through various routes. All administration methods may be expected. For example, azelaic acid may be orally administered, or administered by rectal injection, intravenous injection, intramuscular injection, subcutaneous injection, intrauterine dural injection, or intracerebroventricular injection.

In a case in which the composition according to the present invention is provided as a mixture containing another ingredient added thereto in addition to azelaic acid, the composition may contain azelaic acid in a content of 0.001 wt% to 99.9 wt%, preferably, 0.1 wt% to 99 wt%, and more preferably, 1 wt% to 50 wt% based on a total weight of the composition.

A pharmaceutical administration form of the composition according to the present invention may be a form of a pharmaceutically acceptable salt thereof. Further, the composition may be used alone, or a suitable set as well as a combination of the compound with another pharmaceutically active compound may also be used.

The term "pharmaceutically acceptable salt" means a form of a compound that does not cause severe stimulation in an organism to which the compound is administered and does not damage biological activities and physical properties of the compound. The terms "hydrate", "solvate", and "isomer" have the same meaning as described above. The pharmaceutically acceptable salts may be obtained by reacting the compound containing azelaic acid according to the present invention with an inorganic acid such as hydrochloric acid, bromic acid, sulfuric acid, nitric acid, phosphoric acid, or the like; a sulfonic acid such as methane sulfonic acid, ethane sulfonic acid, p-toluene sulfonic acid, or the like; and an organic carbonic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, salicylic acid, or the like. Further, the pharmaceutically acceptable salts may be obtained by reacting the compound containing azelaic acid according to the present invention with a base to form an alkali metal salt such as an ammonium salt, a sodium salt, a potassium salt, or the like, an alkali earth metal salt such as a calcium salt, a magnesium salt, or the like, a salt of an organic base such as dicyclohexylamine, N-methyl-D-glucamine, tris(hydroxymethyl)methylamine, or the like, and a salt of amino acid such as arginine, lysine, or the like.

The pharmaceutical composition or medicinal composition containing azelaic acid according to the present invention may be formulated in a form for oral administration, such as powders, granules, tablets, capsules, suspensions, emulsions, syrups, aerosols, or the like, for external application, suppository, and sterile injection solutions by general methods, respectively. Examples of the carrier, the excipient, and the diluent capable of being contained in the composition containing azelaic acid may include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, and mineral oil.

In the case in which the pharmaceutical composition is formulated, generally used diluents or excipients such as fillers, extenders, binders, wetting agents, disintegrants, surfactants, or the like, may be used. Solid formulations for oral administration include tablets, pills, powders, granules, capsules, and the like and may be prepared by mixing azelaic acid with at least one excipient, for example, starch, calcium carbonate, sucrose or lactose, gelatin, or the like. Further, lubricants such as magnesium stearate or talc may be used in addition to simple excipients. Liquid formulations for oral administration include suspensions, solutions, emulsions, syrups, and the like, and these liquid formulations may contain various excipients such as a wetting agent, a sweetener, a flavoring agent, a preservative, or the like, as well as water and liquid paraffin that are generally used simple diluents. Formulations for parenteral administration include sterile aqueous solutions, non-aqueous solvents, suspensions, emulsions, freeze-dried formulations, and suppositories. In the non-aqueous solvents or the suspensions, propylene glycol, polyethylene glycol, vegetable oil such as olive oil, injectable esters such as ethyloleate, or the like, may be used. As a base for suppositories, witepsol, macrogol, tween 60, cacao butter, laurinum, glycerol-gelatin, or the like, may be used.

The olfactory receptor is a protein group occupying the largest part of a G-protein coupled receptor (GPCR) protein superfamily, and generally, an expression amount thereof in a membrane is not large. Therefore, the present inventors tried to finally confirm presence or absence of expression (a degree of expression) of the olfactory receptor Olfr544 confirmed in a previous microarray study in the adipose tissue (or 3T3-L1 adipocytes) through RT-PCR, qPCR, western blot and immunohistochemistry experiments.

In an Example of the present invention, expression of Olfr544 gene and protein in the adipose tissue and 3T3-L1 adipocytes of a mouse was confirmed. As a result, it was confirmed that the Olfr544 gene (at an RNA level) was significantly expressed in the adipose tissue and the 3T3-L1 adipocytes in RT-PCR and qPCR experiments as illustrated in FIGS. 2A and 2B. Particularly, in both of the experiments, it was observed that an expression level of Olfr544 was higher in the adipose tissue than the 3T3-L1 adipocyte.

Meanwhile, as illustrated in FIG. 2C, in the western blot experiment, the Olfr544 protein was not detected in the cytosol of the 3T3-L1 adipocyte, but was expressed at a high level in a membrane protein fraction. Further, as illustrated in FIG. 2D, as a result of performing the immunohistochemistry experiment, the Olfr544 protein was significantly expressed in the membrane while being colocalized with a membrane marker in the membrane of the 3T3-L1 adipocyte.

In another Example of the present invention, MTT assay was performed in order to evaluate cytotoxicity of azelaic acid. As a result, it was confirmed that even in a case of treating 3T3-L1 adipocytes with high-concentration azelaic acid (500µM), cytotoxicity was not observed as illustrated in FIG. 3.

In another Example of the present invention, an effect of azelaic acid as an Olfr544 ligand was evaluated. As a result, it was observed that in a case of using cAMP response element (CRE)-luciferase reporter gene assay, azelaic acid concentration-dependently increased an Olfr544 activity in a concentration range of 1 to 1000µM as illustrated in FIG. 4. Further, an EC₅₀ value was 29.71µM, such that about 50% of Olfr544 was activated at this concentration.

In another Example of the present invention, in order to identify whether or not azelaic acid participates in signal transduction by activating Olfr544, changes in concentrations of second messengers in 3T3-L1 adipocytes treated with azelaic acid were confirmed. As a result, it was confirmed that in the 3T3-L1 adipocytes treated with azelaic acid, only a concentration of cAMP was specifically and dependently increased in proportion to a concentration of azelaic acid as illustrated in FIGS. 5A to 5C.

In another Example of the present invention, it was confirmed that azelaic acid specifically increased the concentration of cAMP in the adipose tissue and did not have an influence on concentrations of inositol phosphate and calcium.

Meanwhile, it was known that an increase in cAMP in adipose cells increases a CREB activity and stimulates a PKA activity to induce lipolysis.

In another Example of the present invention, in order to confirm a protein kinase A (PKA) signaling pathway, which is a sub-pathway of cAMP corresponding to the second messenger, a PKA activity regulation effect of azelaic acid was evaluated. As a result, azelaic acid significantly increased the PKA activity in the 3T3-L1 adipocytes as illustrated in FIG. 6. It was confirmed that at the time of treating the 3T3-L1 adipocytes with azelaic acid (50µM) for 2 hours, the PKA activity was increased by 113.8% as compared to a control. Therefore, the PKA activity was increased due to an increase in the adipocytes caused by azelaic acid treatment.

In another Example of the present invention, changes in phosphorylation of cAMP-response element binding protein (CREB) and hormone-sensitive lipase (HSL) protein by azelaic acid were confirmed. As a result, in a case of treating the adipose cells with azelaic acid (50µM) for 2 hours, phosphorylation of CREB and HSL was rapidly increased as compared to a control (C) as illustrated in FIG. 7. An increase in concentration of cAMP in the 3T3-L1 adipocytes stimulates the PKA activity to increase phosphorylation of HSL, and activation of HSL caused by phosphorylation of HSL promotes hydrolysis of triglycerides stored in lipid droplets. Therefore, this result suggests that azelaic acid has an effect of hydrolyzing triglycerides of the adipose cells.

As a result, when Olfr544 corresponding to the olfactory receptor was activated in the adipose tissue (or adipose cells) by azelaic acid corresponding to the ligand, a PKA protein was activated by an increase in concentration of cAMP corresponding to the second messenger, and CREB and HSL corresponding to main targets of the PKA protein were phosphorylated to thereby be activated. Consequently, it was confirmed that activation of Olfr544 caused by azelaic acid treatment activates an intracellular cAMP-CREB signaling pathway.

In another Example of the present invention, in order to confirm that azelaic acid has an effect of activating a cAMP-PKA-HSL signaling pathway to promote triglyceride hydrolysis in the 3T3-L1 adipocytes, concentrations of triglycerides and cholesterol in the adipose cells were measured, and a release amount of glycerol corresponding to a product of triglyceride hydrolysis was measured. As a result, at the time of treating the 3T3-L1 adipocytes with azelaic acid (50µM) for 2 hours, triglycerides in the cells was decreased by 61.5% (P<0.05) and glycerol corresponding to the product of triglyceride hydrolysis was increased by 46.7% as illustrated in FIGS. 8A to 8C. This means that azelaic acid has an effect of promoting triglyceride hydrolysis in the adipose tissue to suppress accumulation of triglycerides.

In another Example of the present invention, a role of Olfr544 in a lipolysis regulation effect of azelaic acid was re-confirmed using Olfr544 shRNA. More specifically, expression of Olfr544 gene was suppressed by transfecting the Olfr544 shRNA into the 3T3-L1 cells corresponding to mouse adipose cells (FIGS. 9A to 9C); at the time of treating cells in which expression of Olfr544 was suppressed with azelaic acid, the cAMP-PKA-HSL signaling pathway was inactivated (FIGS. 10A to 10C), and a change in release concentration of glycerol due to triglyceride hydrolysis in blood caused by azelaic acid treatment was not observed (FIG. 11), such that it was confirmed that lipolysis by azelaic acid was dependent on Olfr544.

Meanwhile, in another Example of the present invention, azelaic acid was orally administered to obesity-induced mouse animal models, and various obesity-associated indices were confirmed. It was observed that in an obesity mouse group to which azelaic acid was administered, there was no change in food intake but an increase in body weight was significantly decreased as compared to a control group (FIG. 12), and total fat, subcutaneous fat, and abdominal fat were significantly decreased as compared to the control group (FIG. 13). Further, as a result of analyzing blood indices, it was confirmed that a concentration of blood cholesterol was significantly decreased by administration of azelaic acid (FIG. 14), but in ob/ob mice fed a high-fat diet, a weight of liver tissue was increased due to generation of fatty liver, but the weight of the liver tissue was decreased by administration of azelaic acid, and side effects such as hepatotoxicity, or the like, was not exhibited (FIG. 15).

Finally, as a result of analyzing energy metabolism depending on administration of azelaic acid in normal mice, in an azelaic acid administration group, there was no change in total energy expenditure (EE) but a respiratory quotient (RQ) was decreased and fatty acid oxidation was increased as compared to a control. Further, there was no large difference in consumption amount of O₂, but a production amount of CO₂ was significantly increased as compared to the control group (FIG. 16).

Taken together, the results of Examples show that a triglyceride hydrolysis promotion effect and a lipid accumulation suppression effect in the adipose tissue (or adipose cells) caused by azelaic acid treatment are resulted from azelaic acid acting as the Olfr544 ligand to activate the intracellular cAMP-PKA-HSL signaling pathway. That is, azelaic acid acts as an agonist of the olfactory receptor Olfr544 expressed in the membrane of the adipose cell to specifically increase the concentration of intracellular cAMP, thereby increasing the PKA activity and phosphorylation of CREB and HSL to promote lipid hydrolysis and induce triglyceride hydrolysis. Therefore, azelaic acid has an effect of decreasing accumulation of triglycerides in the adipose tissue to alleviate obesity and metabolic syndromes.

The compound containing azelaic acid according to the present invention or a pharmaceutically acceptable salt thereof may be used as a main ingredient of food or an additive or adjuvant at the time of preparing various functional foods and health functional foods.

Therefore, the present invention relates to a health functional food for alleviating obesity, containing azelaic acid as an active ingredient.

In the present invention, obesity alleviation may be characterized by a decrease in triglycerides in the adipose tissue, suppression of lipid accumulation in the adipose tissue, a decrease in body weight, or a decrease in body fat.

In the present invention, the term 'functional food' means food obtained by adding azelaic acid to general food to improve functionality of the general food. Functionality may be represented by physical properties and physiological functionality, and in a case of adding azelaic acid according to the present invention to general food, physical properties and physiological functionality of the general food may be improved. In the present invention, food having improved functions as described above is comprehensively defined as the 'functional food'.

The functional food according to the present invention may be variously used in drugs, food, beverages, and the like, for decreasing accumulation of body fat. Examples of the functional food according to the present invention may include foods, candies, chocolates, beverages, gums, teas, vitamin complexes, health supplement foods, and the like, and the functional food may be used in a form of powders, granules, tablets, capsules or beverages.

The composition according to the present invention may be added to food or beverages in order to promote lipolysis in the adipose tissue. In this case, a content of the composition in foods or beverages is as follows. In general, a content of the heat functional food composition according to the present invention may be 0.01 to 50wt%, and preferably 0.1 to 20wt% based on a total weight of food, and a content of a health beverage composition according to the present invention may be 0.02 to 10g, and preferably 0.3 to 1g, based on 100ml of the beverage, but the content is not limited thereto.

There is no particular limitation in liquid ingredients of the health beverage composition according to the present invention as long as the health beverage composition contains the composition according to the present invention as an essential ingredient at the ratio as described, and the health beverage composition may further contain various flavors, natural carbohydrates, or the like, as additional ingredients, similarly to general beverages. Examples of the natural carbohydrates include general sugars, for example, monosaccharides such as glucose, fructose, and the like, disaccharides such as maltose, sucrose, and the like, and polysaccharides such as general sugars such as dextrin, cyclodextrin, and the like, and sugar alcohols such as xylitol, sorbitol, erythritol, and the like. In addition to the above-mentioned ingredients, as the flavor, natural flavors (thaumatin, stevia extracts (for example, rebaudioside A, glycyrrhizin, and the like) and synthetic flavors (saccharine, aspartame, and the like) may be advantageously used. A ratio of the natural carbohydrate in the composition according to the present invention is about 1 to 20g, and preferably about 5 to 12g, based on 100ml of the composition.

Besides the additional ingredients as described above, the composition according to the present invention may contain various nutrients, vitamins, minerals (electrolytes), flavorants such as synthetic flavorants and natural flavorants, colorants and improving agents (cheese, chocolate, and the like), pectic acid and salts thereof, alginic acid and salts thereof, organic acids, protective colloid thickeners, pH control agents, stabilizers, preservatives, glycerin, alcohols, carbonating agents used in a carbonated beverage, or the like. In addition, the composition according to the present invention may contain fruit flesh for preparing natural fruit juices, fruit juice beverages, and vegetable beverages. These ingredients may be used independently or in combination. Although the content of these additives is not particularly important, it is generally selected in a range of 0.01 to 20 parts by weight based on 100 parts by weight of the composition according to the present invention.

Hereinafter, the present invention will be described in detail through the Examples. However, these Examples are only to illustrate the present invention, and those skilled in the art will appreciate that these Examples are not to be construed as limiting a scope of the present invention.

### Example 1: Analysis of Olfr544 Gene and Protein Corresponding to Molecular Targets of Azelaic Acid

In order to confirm expression of an olfactory receptor Olfr544 (hereinafter, referred to Olfr544) protein corresponding to a molecular target of azelaic acid in adipose cells, an experiment for measuring expression of gene and protein was performed (FIG. 2). Expression of Olfr544 genes in the adipose tissue of a mouse and differentiated and cultured 3T3-L1 adipocytes were confirmed using RT-PCR and quantitative real time (qPCR) experiments, respectively, and expression of Olfr544 protein in the cells was confirmed through western blot and immunohistochemistry experiments.

### 1-1: Cell Culture

Adipose cells used in Examples 1 to 7, which were obtained by differentiating 3T3-L1 preadipocytes (KCLB No. 10092.1), were obtained from Korean Cell Line Bank, and these cells were sub-cultured and maintained when the cells grew at a confluency (degree of proliferation) of 50% in a Dulbecco's modified Eagle's medium (DMEM) containing 10% fetal calf serum (FCS) at 37°C under 5% CO₂ conditions (Green H., et al., Cell, 3(2):127-33, 1974; Green H., et al., Cell, 5(1) :19-27, 1975; Atanasov, A. G., et al., Biochimica et Biophysica Acta(BBA)-General Subjects 1830(10):4813-9).

### 1-2: Cell Differentiation

A differentiation process of the 3T3-L1 preadipocytes was as follows. First, 1 x 10⁶ 3T3-L1 preadipocytes were seeded in each well of a 6-well plate and cultured so as to have a confluency of 100%. After 2 days, the cultured 3T3-L1 preadipocytes were treated in a DMEM medium containing 10% fetal bovine serum (FBS) and a MDI solution (0.5mM IBMX, 0.5µM dexamethasone, and 10µg/mL insulin) for 3 days. Next, the treated cells were cultured in the DMEM containing 10% FBS and 10µg/mL insulin, presence or absence (degree) of a lipid droplet in the cells was confirmed, and based on the confirmation results, adipocytes were differentiated (Green H., et al., Cell, 3(2):127-33, 1974; Green H., et al., Cell, 5(1):19-27, 1975; Atanasov, A. G., et al., Biochimica et Biophysica Acta(BBA)-General Subjects 1830(10):4813-9).

As a result, it was confirmed that about at least 80% of the 3T3-L1 preadipocytes were differentiated into the adipocytes through a differentiation process for a total of 12 days (data were not illustrated).

### 1-3: Expression of Olfr544 Gene

In order to confirm RNA expression of Olfr544 genes, RT-PCR and qRT-PCR were performed.

### (1) Synthesis of cDNA

First, RNA was extracted from the 3T3-L1 adipocytes differentiated by the methods in Examples 1-1 and 1-2 using a method known to those skilled in the art, and cDNA was synthesized using the extracted RNA and a ReverTra Ace® qPCR RT kit (TOYOBO, Osaka, Japan). More specifically, in order to improve efficiency of a RT-PCR reaction, the extracted RNA was treated at 65°C for 5 minutes and then directly stored in ice. Thereafter, a total of 8µl of reactants were prepared using 2µl of 4x DNA Master Mix containing a gDNA remover, 0.5µg of RNA, and distilled water (nuclease-free water) and reacted at 37°C for 5 minutes. Then, 5x RT Master mix was added to the reaction product, and a reaction was carried out at 37°C for 5 minutes, at 50°C for 5 minutes, and at 98°C for 5 minutes, thereby synthesizing cDNA.

### (2) PCR

A polymerase chain reaction (PCR) was performed on the cDNA synthesized by the method in (1) using a primer illustrated in Table 1 by a method known to those skilled in the art. Next, the obtained PCR product was electrophoresed on agarose gel, and whether or not RNA of the Olfr544 gene was expressed was confirmed through a band. Here, as a control, glyceraldehyde-3-phosphate dehydrogenase (GAPDH, a sequence generally known to those skilled in the art) was used.

The following Table 1 illustrates an Olfr544 primer sequence.

**[Table 1]**

| **name** | **type** | **Sequence (5'->3')** | **Seq. NO** |
|---|---|---|---|
| mOlfr544_F | sense | GGG GAC ATC TCG CTG AAT AA | 1 |
| mOlfr544_R | anti-sense | ATG AGG ACA TGG TGG AGG AG | 2 |

### (3) qPCR(quantitative real-time PCR)

qPCR was performed using the cDNA synthesized in the method in (1), the primer illustrated in Table 1, and a Thunderbird Sybr qPCR Mix (TOYOBO, Osaka, Japan) and an iQ5 iCycler system (Bio-Rad, California, U.S.) by a method known to those skilled in the art. More specifically, a denaturation process was performed at 95°C for 4 minutes and 30 seconds, a denaturation process was additionally performed for 40 cycles at the same temperature for 10 seconds, an annealing process was performed at 55°C to 60°C for 30 seconds, and then, an extension process was performed at 68°C for 20 seconds. A degree of expression of each of the genes was standardized using expression of GAPDH. A primer was designed using a nucleotide blast software of National Center for Biotechnology Information (NCBI) and purchased from Bionics (Seoul, Korea).

As a result, it was confirmed that the Olfr544 gene (at an RNA level) was significantly expressed in the adipose tissue of the mouse and the 3T3-L1 adipocytes in the RT-PCR and qPCR experiments as illustrated in FIGS. 2A and 2B. Particularly, in both of the experiments, it was observed that an expression level of Olfr544 was higher in the adipose tissue than the 3T3-L1 adipocytes.

### 1-4: Expression of Olfr544 Protein

In order to confirm expression of a protein corresponding to a final product of the Olfr544 gene, protein elecrotrophoresis (sodium dodecyl sulfate-acrylamide gel electrophoresis (SDS-PAGE)), western blot, and immunohistochemistry experiments were performed.

### (1) Protein Extraction

Proteins were extracted from the 3T3-L1 adipocytes differentiated by the methods in Examples 1-1 and 1-2.

In a case of obtaining a membrane protein fraction, the 3T3-L1 adipocytes (or the adipose tissue) were rinsed with phosphate-buffered saline (PBS) to collect only cell (or tissue) pellets and washed with a C solution (120mM NaCl, 5mM KCl, 1.6mM MgSO₄, 25mM NaHCO₃, 7.5mM D-glucose, pH 7.4), and then, a solution D (the solution C + 10mM CaCl₂) was added thereto and stirred for 20 minutes, followed by centrifugation. A precipitate obtained by performing centrifugation of the supernatant obtained after centrifugation again was dissolved in a TEM buffer solution (10mM Tris, 3mM MgCl₂, 2mM EDTA, pH 8.0) and glycerol, thereby finally obtaining the membrane protein fraction. A protein extraction method as described above is known as a calcium ion shock method in which a protein yield is higher than that in a mechanical stirring method corresponding to an existing membrane fractionation method.

Meanwhile, in a case of obtaining a cytosol protein fraction, the 3T3-L1 adipocytes (or the adipose tissue) were rinsed with PBS to collect only the pellets, a digitonin-contained buffer solution (150mM NaCl, 50mM HEPES, 25µg/ml digitonin, pH 7.4) was added thereto, and the resultant was allowed to stand on ice for 10 minutes, followed by centrifugation, thereby finally obtaining a supernatant as the cytosol protein fraction.

Meanwhile, in a case of obtaining whole protein extract without a fractionation process, a cell lysis buffer solution (50mM Tris, 1% Triton-X 100, 1mM EDTA, proteinase inhibitor (PI)) was added to the 3T3-L1 adipocytes to perform cell lysis, followed by centrifugation at 13,000 rpm and at 4°C for 20 minutes, thereby obtaining a supernatant.

### (2) Protein Electrophoresis (SDS-PAGE) and Western Blot

In order to confirm whether or not the Olfr544 protein was expressed, the protein fractions (membrane protein, cytosol protein, and whole protein extract) extracted in the method in (1) were quantified by a Bradford method. 12% SDS-PAGE was performed using a denaturized protein obtained by extracting about 40µg of protein from each test sample and suitably heating and/or dissolving the extracted protein depending on the type of extracted protein (fractionated protein, whole protein, or phosphorylated protein, or the like).

At the time of performing the western blot experiment, in order to prevent non-specific binding to a nitrocellulose (NC) membrane containing a protein blotted from a SDS-PAGE gel, after blocking, the protein was sequentially treated with a primary antibody (anti-Olfr544 rabbit antibody (Abcam, U.K.)) for a protein to be detected, anti-β-actin antibody (loading control), (Santa Cruz, California, U.S.) and a secondary antibody (HRP-conjugated-anti-rabbit IgG, Santa Cruz, California, U.S.), and treated at room temperature for 1 hour, respectively. Then, for content comparison, contents of the protein bands in the western blot experiment were quantified and numeralized by a software program (Gel-Pro Analyzer 4.0).

### (3) Immunohistochemistry

In order to confirm a degree of intracellular expression and an expression position of Olfr544 through immunohistochemistry, after the cultured cells were seeded in a 6-well plate at a concentration of 1 x 10⁵ cells/well and attached and stabilized for 24 hours, a lucy-flag-Olfr544 expression vector was transfected into the 3T3-L1 adipocytes using Lipofectamine 2000 (Invitrogen, California, U.S.). Next, the cells were washed with PBS, and treated with 4% para-formaldehyde for 20 minutes, thereby immobilizing the 3T3-L1 adipocytes transfected with the lucy-flag-Olfr544 expression vector, and Olfr544 proteins, membranes, and nucleus were stained with respective dyes (i, ii, iii) to be described below.
i) In order to stain the Olfr544 protein, after the 3T3-L1 adipocytes were reacted with an anti-flag primary antibody (Sigma, U.S.) or anti-Olfr544 antibody (Abcam, Cambridge, U.K.) at room temperature for 1 hour and washed with PBS three times, an anti-rabbit IgG secondary antibody labeled with green fluorescence was added thereto, and light was blocked using foil, followed by treatment for 1 hour.
ii) In order to stain the membrane, the membrane was treated with fluorescence-labeled CellMask™ Orange Plasma Membrane Stains (Invitrogen, California, U.S.) at 37°C for 10 minutes, thereby staining the membrane.
iii) In order to stain the nucleus, after the nucleus was treated with a 4',6-diamidino-2-phenylindole (DAPI) solution (Sigma, U.S.) to thereby be stained, a cover slide was covered thereon to airtightly seal the stained nucleus.

The finally stained 3T3-L1 adipocytes as described above were photographed using a confocal fluorescent microscope (LSM700, Carl Zeiss).

As a result, as illustrated in FIG. 2C, in the western blot experiment, the Olfr544 protein was not detected in the cytosol of the 3T3-L1 adipocytes, but was expressed at a high level in the membrane protein fraction. Further, as illustrated in FIG. 2D, as a result of performing the immunohistochemistry experiment, the Olfr544 protein was significantly expressed in the membrane while being colocalized with a membrane marker in the membrane of the 3T3-L1 adipocyte.

### Example 2: Evaluation of Cytotoxicity of Azelaic Acid

In order to evaluate cytotoxicity of azelaic acid, MTT assay was performed.

The MTT assay is a test method using an ability of mitochondria reducing MTT tetrazolium, a yellow watersoluble substrate, to a blue purple water-insoluble MTT formazane (3-(4,5-dimethylthiazol-2-yl)-2,5-diphenyltetrazolium bromide). An MTT reagent was prepared by diluting C₁₈H₁₆BrN₅S (Thiazoly Blue Tetrazolium Bromide) in phosphate buffered saline (PBS) at a concentration of 2 to 5mg/ml.

First, after the 3T3-L1 adipocytes (4×10⁵ cells/ml) were seeded in a 96-well plate and cultured at 37°C under 5% CO₂ conditions for 24 hours, the cultured 3T3-L1 adipocytes were treated with azelaic acid in a concentration range of 0 to 500µM and cultured for 24 hours. After 100µL of the MTT reagent was added to each test sample at a concentration of 4mg/mL and cultured at 37°C under 5% CO₂ conditions for 4 hours, 100µL of dimethyl sulfoxide (DMSO) was added thereto, and absorbance was measured at 540nm. An absorbance value is to quantify an apoptotic effect due to toxicity using the principle that absorbance is in proportion to the number of living cells.

As a result, it was confirmed that even in a case of treating the 3T3-L1 adipocytes with high-concentration azelaic acid (500µM), cytotoxicity was not observed as illustrated in FIG. 3. Therefore, it may be appreciated that azelaic acid contained in various ingestible plants including grains such as barley, oats, and the like, and formed in a metabolic process in a human body to thereby be present in a concentration range of about 10∼50µM is a material which may be safely ingested.

### Example 3: Evaluation of Effect of Azelaic Acid as Olfr544 Ligand

In order to evaluate an activity of azelaic acid as an Olfr544 ligand in vitro assay, reporter gene assay was performed. Generally, a degree of gene expression of the olfactory receptor (OR) in tissue cells was low and a degree of protein expression also was low, such that research into the olfactory receptor was difficult. In order to solve this problem, a Hana3A cell line was supplied from a team led by professor Hiroaki Matsunami of Duke University and used in an experiment (Zhuang H., et al., Nat. Protocol, 3:1402-1413, 2008). The Hana3A cell line, which is a stable cell line obtained by transforming a HEK293T cell line so as to co-express a receptor-transporting protein 1S (RTP1S) and a RIC8 Guanine Nucleotide Exchange Factor B (Ric8b) which were cofactors essential in membrane expression of an OR protein, is a cell line significantly improving (optimizing) membrane expression of the OR protein.

The reporter gene assay was performed as follows. First, after an Olfr544 expression vector and a cAMP response element (CRE)-Luciferase reporter vector were transfected into the Hana3A cell line, the transfected Hana3A cell line was treated with azelaic acid for 18 hours. Then, a luciferase activity was measured using a Firefly luciferase (FL) assay kit, and a dose response curve (DRC) was drawn based on the measured luciferase activity, thereby calculating a 50% effective concentration (EC₅₀) corresponding to an index for determining an effect as a ligand (agonist).

As a result, it was observed that in a case of using cAMP response element (CRE)-luciferase reporter gene assay, azelaic acid concentration-dependently increased an Olfr544 activity in a concentration range of 1 to 1000µM as illustrated in FIG. 4. Further, the EC₅₀ value was 29.71µM, such that about 50% of Olfr544 was activated at this concentration. Azelaic acid is naturally formed in the body, and generally, a blood concentration of azelaic acid has been variously reported at several tens to several hundreds micromolar (µM) levels, such that the measured EC_{5C} value indicates that azelaic acid may activate Olfr544 in the body without toxicity.

Consequently, an effective administration dose of azelaic acid was calculated based on the EC₅₀ value (29.71µM) of azelaic acid with respect to Olfr544 corresponding to a target protein of azelaic acid, confirmed in FIG. 4 and EC₅₀ values of Actos® (pioglitazone, PPAR-γ agonist, EC_{5C}=0.5µM, human administration dose: 15∼30mg/day) corresponding to a drug for metabolic diseases and Xenical® (orlistat, pancreatic lipase inhibitor, EC_{5C}=0.24µM, human administration dose: 120mg/day), the effective administration dose of azelaic acid is about 900 to 1800mg/day. This is a dose capable of being used as a daily intake amount, and considering an amount of azelaic acid formed in the body and an amount of azelaic acid ingested from general food, actually, an effective intake amount of azelaic acid may be further decreased.

### Example 4: Analysis of Change in Concentration of Second messengers in 3T3-L1 Adipocytes Treated with Azelaic Acid

The present inventors tried to confirm whether or not azelaic acid activates Olfr544 corresponding to a G-protein coupled receptor (GPCR) and participates in signal transduction associated with triglyceride hydrolysis.

GPCR was known to activate G-proteins by ligand binding to thereby regulate intracellular signal transduction using a second messenger. Depending on the kind of G-protein, cAMP, IP₃, and/or calcium were used as the second messengers.

Therefore, changes in concentrations of cAMP, IP-one (metabolite of IP₃), and calcium, which are second messengers by azelaic acid corresponding to the Olfr544 ligand, in 3T3-L1 adipocytes were measured. Since IP₃ has low stability to thereby be easily decomposed to IP-one, an amount of intracellular IP-One (metabolite of IP₃) was quantified instead of IP₃.

cAMP assay uses the principle to estimate a production amount of cAMP corresponding to a second messenger of a GPCR receptor activated by a ligand using a cAMP standard calibration curve. A 96-well plate included in a cAMP assay ELISA kit (Enzo Life science, New York, U.S.) was coated with a GxR IgG antibody, and after cAMP of a blue solution for obtaining the standard calibration curve was bound to alkaline phosphatase, the color of the blue solution was changed to yellow by a rabbit antibody. When alkaline phosphatase was activated by adding a pNpp substrate to a well bound to cAMP, a color change occurred, such that the concentration of cAMP may be measured at a wavelength of 405nm using colorimetric analysis. In the present experiment, 1 x 10⁴ 3T3-L1 adipocytes were seeded in each well of the 96-well plate and differentiated, and then treated with 50µM azelaic acid and 1µM Foskolin corresponding to a positive control for 18 hours. Then, the concentration of cAMP was measured by the method described above.

IP-one assay is a method of measuring a concentration of IP₃ corresponding to a second messenger formed by phospholipolysis by phospholipase in a GPCR signal transduction process to measure an ability to regulate protein kinase C (PKC) signal transduction. In the present experiment, 1 x 10⁴ 3T3-L1 adipocytes were seeded in each well of the 96-well plate and differentiated, and then treated with 50µM azelaic acid for 18 hours. Then, in order to measure IP₃, the concentration of intracellular IP-one (metabolite of IP₃, a surrogate marker) of which an error was small was measured using an IP-one ELISA Assay kit (Cisbio, France).

The concentration of intracellular calcium was measured using a Fluo-4 Direct™ Calcium Assay Kit (Invitrogen, California, U.S.) containing a fluorescent dye for labeling calcium ions. The cultured 3T3-L1 adipocytes were obtained and subjected to centrifugation, and then only pellets were diluted in a Fluo-4 Direct™ calcium assay buffer solution contained in the kit at a concentration of 2.5 x 10⁶ cells/mL. After the resultant was seeded in a 96-well plate and cultured at 37°C under 5% CO₂ conditions for 60 minutes, 50µL of a 2X Fluo-4 Direct™ calcium reagent loading solution was seeded in the 96-well plate and cultured at 37°C under 5% CO₂ conditions for 30 minutes. Then, fluorescence was measured at excitation and emission wavelengths of 494nm and 516nm.

As a result, azelaic acid significantly increased the concentration of intracellular cAMP but did not affect the concentrations of IP₃ and calcium as illustrated in FIGS. 5A to 5C. An increase in cAMP concentration by azelaic acid tended to increase depending on a concentration of azelaic acid. Therefore, it may be confirmed that azelaic acid specifically increases concentration of intracellular cAMP by activating Olfr544 in the 3T3-L1 adipocytes.

### Example 5: Evaluation of PKA Activity Regulation Effect of Azelaic Acid

A representative protein regulating protein activities associated with signal transduction depending on a change in concentration of intracellular cAMP in the tissue cells is protein kinase A (PKA). Under general conditions, since an enzyme active site is blocked by a regulatory domain site of protein, a PKA activity is suppressed, but when the concentration of cAMP is increased, cAMP is bound to a regulatory site of PKA to expose an active site, such that the PKA activity is increased. In the present experiment, a change in PKA activity by azelaic acid was measured. The PKA activity was measured using an Enzo PKA kinase activity kit (Cat. # ADI-EKS-390A, Enzo Life Sciences, Korea).

As a result, azelaic acid significantly increased the PKA activity in the 3T3-L1 adipocytes as illustrated in FIG. 6. It was confirmed that at the time of treating the 3T3-L1 adipocytes with azelaic acid (50µM) for 2 hours, the PKA activity was increased by 113.8% as compared to a control. Therefore, the PKA activity was increased due to the increase in cAMP in the adipocytes caused by azelaic acid treatment.

### Example 6: Analysis of Changes in Phosphorylation of CREB and HSL Protein by Azelaic Acid

When the PKA protein activity is increased by azelaic acid, phosphorylation of target proteins of PKA is increased. Therefore, phosphorylation of cAMP-response element binding protein (CREB) and hormone-sensitive lipase (HSL) known as target proteins of PKA was confirmed by a western blot experiment. In the western blot experiment, the method described in Example 1 was used, and after azelaic acid was reacted with 3T3-L1 adipocytes for 2 hours, proteins were extracted, thereby performing the experiment. Primary antibodies used in the experiment were anti-phospho-CREB and anti-phospho-HSL antibodies (Santa Cruz, California, U.S.).

As a result, in a case of treating the adipocytes with azelaic acid (50µM) for 2 hours, phosphorylation of CREB and HSL was rapidly increased as compared to a control (C) as illustrated in FIG. 7. An increase in concentration of cAMP in adipocytes stimulates the PKA activity to increase phosphorylation of HSL, and activation of HSL caused by phosphorylation of HSL promotes hydrolysis of triglycerides stored in lipid droplets. Therefore, this result suggests that azelaic acid has an effect of hydrolyzing triglycerides of the adipocytes.

### Example 7: Analysis of Change in Concentrations of Triglyceride and Cholesterol and Lipid Hydrolysis in Adipocytes by Azelaic Acid

In order to confirm whether or not azelaic acid induces triglyceride hydrolysis in adipocytes by activating a cAMP-PKA-HSL signaling pathway, the adipocytes were directly treated with azelaic acid, and concentrations of intracellular lipids were measured.

After treating 3T3-L1 adipocytes with azelaic acid (concentration: 25 or 50µM), intracellular lipids were extracted. Then, a concentration of triglycerides was measured using a TG assay kit (BioVision, California, U.S.) and a concentration of cholesterol was measured using an Amplex Red cholesterol assay kit (Invitrogen, California, U.S.) .

As a result, azelaic acid significantly and concentration-dependently decreased the concentration of triglycerides in the 3T3-L1 adipocytes as illustrated in FIGS. 8A to 8C. In a case of treating the 3T3-L1 adipocytes with 50µM azelaic acid, the concentration of intracellular triglycerides was decreased by 61.5% as compared to a control. Meanwhile, there was no significant change in the concentration of intracellular cholesterol, such that it may be appreciated that azelaic acid selectively hydrolyzed the triglycerides in the adipose tissue.

Further, in order to confirm whether or not azelaic acid has an effect of hydrolyzing triglycerides, a release amount of glycerol corresponding to a product of triglyceride hydrolysis was measured. As a result of treating the 3T3-L1 adipocytes with 50µM azelaic acid for 2 hours, glycerol corresponding to the product of triglyceride hydrolysis was increased by 46.7% as compared to the control, such that the release amount of glycerol was significantly increased.

Taken together, it was confirmed that azelaic acid according to the present invention was bound to Olfr544 corresponding to the GPCR expressed in the membranes of the adipocytes as a ligand to activate Olfr544 and activate the cAMP-PKA-HSL signaling pathway, thereby hydrolyzing triglycerides to decrease the concentration of triglycerides in the adipocytes. Particularly, since azelaic acid, which is a natural material naturally formed in the body, is contained in grains such as barley, rye, and the like, azelaic acid is an ingestible ingredient, and acts on adipocytes in the body to promote triglyceride hydrolysis, thereby making it possible to obtain a body weight decreasing effect and an anti-obesity effect.

### Example 8: Confirmation of Olfr544 Dependency in Lipolysis Regulation Effect of Azelaic Acid using Olfr544 shRNA

### 8-1: Knock-down of Olfr544 Gene in Adipocytes using Olfr544 shRNA

In order to confirm a knock-down effect of Olfr544 genes in mouse adipocytes, 3T3-L1 cells were differentiated into adipocytes in a 6-well plate. After a shRNA base sequence (top strand: 5'-CACCGCTCACTGTTCGCATCTTCATTCGAAAATGAAGATGCGA-ACAGTGAG-3') targeting Olfr544 or encoding non-targeting scrambled shRNA hairpins (top strand: 5'-CACCGTAAGGCT-ATGAAGAGATACCGAAGTATCTCTTCATAGCCTTA-3') was inserted into a pENTR/U6 vector using a Block-iT U6 RNAi entry vector kit (Invitrogen) to construct Olfr544 shRNA, 2.5µg of Olfr544 shRNA or scrambled shRNA was transfected using 10µL of Lipofectamin2000 (Invitrogen). After 48 hours, expression of Olfr544 was measured. Expression patterns of Olfr544 RNA and proteins were confirmed by the methods described in Examples 1-3 and 1-4. Scr shRNA was used as a negative control.

As a result, it may be confirmed that expression of Olfr544 RNA and proteins was decreased by 80% and 50%, respectively, by the Olfr544 shRNA as illustrated in FIGS. 9A to 9C.

### 8-2: Effect of Inactivating Signal Transduction of Azelaic acid in Adipocyte in Which Olfr544 Gene Was Knocked-down

After the Olfr544 gene was knocked-down in the mouse adipocytes, cAMP assay was confirmed by the method described in Example 4, PKA activity was confirmed by the method described in Example 5, and phosphorylation of HSL protein was confirmed by the method described in Example 6.

As a result, it may be confirmed that in the adipocytes in which the Olfr544 shRNA was transfected and thus, expression of the Olfr544 gene was inhibited, even though the adipocytes were treated with azelaic acid, the cAMP-PKA-HSL signaling pathway was all inactivated as illustrated in FIGS. 10A to 10C.

### 8-3: Measurement of Lipolysis Effect of Azelaic acid in Adipocyte in Which Olfr544 Gene Was Knocked-down

After the Olfr544 gene was knocked-down in the mouse adipocytes, the adipocytes were treated with azelaic acid, and whether or not a triglyceride hydrolysis effect of azelaic acid was maintained was confirmed.

As a result, in the adipocytes in which the Olfr544 shRNA was transfected and thus, expression of the Olfr544 gene was inhibited, even though the adipocytes were treated with azelaic acid, there was no change in concentration of glycerol, as illustrated in FIG. 11. Therefore, it may be confirmed that lipolysis by azelaic acid in the adipocytes was dependent on Olfr544.

### Example 9: Confirmation of Anti-obesity Effect of Azelaic Acid Using Obesity-Induced Mouse Animal Model

After orally administering azelaic acid to an *ob*/*ob* mouse (Samtako, Seoul, Korea) corresponding to a genetically obesity-induced mouse at a concentration of 50mg/kg, a degree of obesity thereof was compared with that of a control group to which azelaic acid was not administered. More specifically, an increase in body weight, a feed intake amount, body fat, blood indices, blood lipid indices, a weight of liver tissue, ALT and AST corresponding to hepatotoxicity indices, and energy metabolism were analyzed.

### 9-1: Analysis of Increase in Body Weight and Feed Intake Amount Depending on Administration of Azelaic Acid

Changes in body weight in azelaic acid administration group and a control group were observed once a week for an experiment period of 6 weeks.

As a result, it may be confirmed that a body weight of the azelaic acid administration group was significantly decreased on and after the first week of the experiment as compared to the control group as illustrated in FIGS. 12A and 12B. Meanwhile, as a result of measuring the feed intake amount at a final week of the experiment, there was no significant difference between the azelaic acid administration group and the control group.

### 9-2: Analysis of Body Fat Depending on Administration of Azelaic Acid

Body fats (total fat, subcutaneous fat, and abdominal fat) of mice in the azelaic acid administration group to which azelaic acid was orally administered for 6 weeks and a control group were measured through micro CT image analysis. The subcutaneous fat was quantified for the entire site of the mouse, and the abdominal fat was calculated by quantifying an amount of abdominal fat at the 15^{th} to 20^{th} lumbar spine sites of the mouse.

As a result, it may be confirmed that the total fat, the subcutaneous fat, and the abdominal fat of the mouse to which azelaic acid was orally administered for 6 weeks were significantly decreased as compared to the control group as illustrated in FIG. 13.

### 9-3: Analysis of Blood Lipid Indices Depending on Administration of Azelaic Acid

In order to evaluate an effect on mouse lipid metabolism of azelaic acid in the administration group to which azelaic acid was orally administered for 6 weeks and the control group, concentrations of blood triglycerides, adiponectin, and cholesterol were measured.

As a result, it was confirmed that in the azelaic acid administration group, the concentrations of blood triglyceride and cholesterol were significantly decreased as illustrated in FIG. 14.

### 9-4: Analysis of Weight of Liver Tissue, ALT, and AST Depending on Administration of Azelaic Acid

Weights of the liver tissue, ALT, and AST of the mice in the azelaic acid administration group to which azelaic acid was orally administered for 6 weeks and the control group were measured.

As a result, the weight of the liver tissue was decreased in the azelaic acid administration group, but there was no large difference in ALT and AST corresponding to indices indicating hepatotoxicity between the azelaic acid administration group and the control group as illustrated in FIGS. 15A to 15C.

### Example 10: Analysis of Energy Metabolism Depending on Administration of Azelaic Acid in Normal Mouse

Relative metabolic rates were confirmed by measuring consumption amount of O₂ and production amounts of CO₂ of an azelaic acid administration group and a control group using indirect calorimetry.

As a result, it may be confirmed that in the azelaic acid administration group, there was no change in total energy expenditure (EE), but a respiratory quotient (RQ) was decreased, and fatty acid oxidation was increased as illustrated in FIG. 16. A decrease in respiratory quotient means that fatty acid is used as a main energy source. Therefore, it may be appreciated from the above-mentioned result that azelaic acid caused lipolysis of the adipose tissue to increase a ratio of fat consumed as an energy source, and thus, an amount of stored fat was decreased and body fat was decreased.

### [Industrial Applicability]

A composition containing azelaic acid, according to the present invention, has an excellent effect of reducing accumulation of lipids in adipose tissue and improving lipid metabolism in the adipose tissue, such that the composition may be usable as a food material, a pharmaceutical composition, and a health functional food for improving the lipid metabolism of the adipose tissue (obesity alleviation) such as body weight reduction and body fat reduction.
## Claims

1. A pharmaceutical composition for use in treating obesity in an obese subject, which contains azelaic acid as an active ingredient.

2. The pharmaceutical composition for use according to claim 1, wherein the azelaic acid promotes triglyceride hydrolysis in the adipose tissue.

3. A health functional food for use in alleviating obesity in an obese subject, which contains azelaic acid as an active ingredient.

## Patentansprüche

1. Pharmazeutische Zusammensetzung zur Verwendung bei der Behandlung von Adipositas bei einem adipösen Patienten, die Azelainsäure als Wirkstoff enthält.

2. Pharmazeutische Zusammensetzung zur Verwendung gemäß Anspruch 1, wobei die Azelainsäure die Triglyceridhydrolyse im Fettgewebe fördert.

3. Funktionelles Lebensmittel zur Verwendung bei der Linderung von Adipositas bei einem adipösen Patienten, das Azelainsäure als Wirkstoff enthält.

## Revendications

1. Composition pharmaceutique à utiliser dans le traitement de l'obésité chez un patient obèse, contenant de l'acide azélaïque comme principe actif.

2. Composition pharmaceutique à utiliser selon la revendication 1, dans laquelle ledit acide azélaïque promeut l'hydrolyse des triglycérides dans le tissu adipeux.

3. Aliment fonctionnel à utiliser dans le soulagement de l'obésité chez un patient obèse, contenant de l'acide azélaïque comme principe actif.


<110> KOREA UNIVERSITY RESEARCH AND BUSINESS FOUNDATION
<120> Composition of Azelaic Acid Having Adipose Triglyceride Hydrolysis effect
<130> PP-B1736
<150> KR 10-2015-0095529
   <151> 2015-07-03
<160> 2
<170> KopatentIn 2.0
<210> 1
   <211> 20
   <212> DNA
   <213> Artificial Sequence
<220>
   <223> m olfr544_F
<400> 1
   ggggacatct cgctgaataa 20
<210> 2
   <211> 20
   <212> DNA
   <213> Artificial Sequence
<220>
   <223> m olfr544_R
<400> 2
   atgaggacat ggtggaggag 20